(19) Europäisches Patentamt
European Patent Office
Office européen des brevets

(11) Publication number: 0 106 193
A2

# (12) EUROPEAN PATENT APPLICATION

(21) Application number: 83109292.9

(22) Date of filing: 19.09.83

(51) Int. Cl.³: A 61 K 7/50
C 11 D 3/37

(30) Priority: 20.09.82 US 420337

(43) Date of publication of application:
25.04.84 Bulletin 84/17

(84) Designated Contracting States:
AT BE CH DE FR GB IT LI LU NL SE

(71) Applicant: UNION CARBIDE CORPORATION
Old Ridgebury Road
Danbury Connecticut 06817(US)

(72) Inventor: Turney, Mary Elizabeth
151 South State Road
Briarcliff Manor New York 10510(US)

(74) Representative: Schmied-Kowarzik, Volker, Dr. et al,
Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8
D-8000 München 40(DE)

## (54) Liquid skin cleanser composition.

(57) A liquid skin cleanser composition comprising (a) a synthetic detergent, (b) a water soluble cationic polymer, (c) a fatty acid soap, and (d) water.

EP 0 106 193 A2

BACKGROUND OF THE INVENTION

This invention is directed to a liquid skin cleanser composition comprising: (a) a synthetic detergent, (b) a fatty acid soap, (c) a water soluble cationic polymer, and (d) water.

The cleaning of skin with liquid cleaning preparations has grown in popularity. Skin cleansers should cleanse the skin gently, causing little or no irritation, without defatting and drying the skin after frequent routine use. These cleansers generally contain mild synthetic detergents, foam builders, and optional ingredients such as perfumes, opacifiers, etc. However, in these formulations, mildness was often obtained at the expense of effective cleaning and good lathering. The addition of fatty acid soaps to the formulations could impart enhanced cleaning and lathering properties. However, at their naturally high pH they would impart undesirable harshness properties to the formulations.

Therefore, there is a desire to formulate a liquid skin cleanser which contains soap yet is mild and has good cleaning and lathering properties.

DESCRIPTION OF THE INVENTION

It has now been found that the addition of a water soluble cationic polymer to a liquid cleansing composition including a fatty acid soap imparts the desired mildness properties while retaining the cleaning and lathering characteristics of the formulation.

Also, it has been found that the incorporation of a water soluble cationic polymer to such a composition including the soap allows the pH of the composition to be lowered such that it approximates the pH of the skin. This results in a cleanser which exhibits especially creamy lathering properties as well as one which imparts a perceptible improvement in skin feel. An added benefit is that at the lower pH the natural antimicrobal properties of the fatty acids may be seen.

The liquid skin cleanser composition of this invention comprises:

(a) a synthetic detergent,

(b) a fatty acid soap,

(c) a water soluble cationic polymer, and

(d) water.

The synthetic detergent suitable for use herein is preferably a non-soap anionic surfactant. These include water soluble salts of organic sulfuric reaction products having in their molecular structure an alkyl group containing from about 8 to about 20 carbon atoms, and a radical selected from the group consisting of sulfuric acid ester and sulfonic acid radicals. Examples of this type of non-soap anionic surfactants include the sodium or potassium alkyl sulfates, especially those derived by sulfation of higher alcohols produced by reduction of tallow or coconut oil glycerides; sodium or ammonium α-olefin sulfonates; sodium or potassium alkyl benzene sulfonates, especially those of the types described in U.S. Patents 2,220,099 and 2,477,383 in which the alkyl group contains from about 9 to about 15 carbon atoms; sodium alkylglyceryl ether sulfonates, especially those ethers of higher alcohols obtained from tallow and coconut oil; sodium coconut oil fatty acid monoglyceride sulfates and sulfonates; sodium salts of sulfuric acid esters of the reaction product of one mole of a higher fatty alcohol (i.e., tallow or coconut oil alcohols) and about 3 moles of ethylene oxide; and others well known in the art, a number being specifically set forth in U.S. Patent Nos. 2,486,921 and 2,486,922.

Additional non-soap anionic organic synthetic surfactants which can be used in this invention include the salts of the condensation products of fatty acids with sarcosin, i.e. acyl sarcosinate, wherein the acyl radical has a chain length range from about 10 to 18 carbon atoms.

The preferred surfactants are the alkali metal (e.g., sodium or potassium) and ammonium, alkyl sulfates derived by sulfation of $C_8$ to $C_{22}$ alcohols, either synthetically derived or produced by reduction of glycerides of tallow or coconut oil; and the alkali metal and ammonium salts of the sulfuric acid esters of the reaction product of 1 mole of a $C_8$ to $C_{22}$ alcohol (e.g., tallow or coconut oil alcohols) and about 1 to 20, preferably 1 to 13, moles of ethylene oxide (ethoxy groups).

The preferred surfactants for use in the present compositions include ammonium or sodium lauryl sulfate and ammonium or sodium lauryl ether sulfate, or combinations thereof.

Also, compatible amounts of nonionic, amphoteric, and cationic synthetic detergents may be included as optional ingredients.

The fatty acid soaps which may be used are well known in the art and include the sodium, potassium and lower alkanolamine salts of naturally occuring vegetable or animal fats and oils. For example, sodium, potassium and triethanolamine salts of fatty acids occuring in coconut oil, soybean oils, castor oil, tallow or synthetically produced fatty acids may be used.

The cationic polymers usefully employed in the compositions of the present invention belong to a class of well known compounds. In a general fashion this term "cationic polymers" means those polymers exhibiting in their principal chain or in the substituted form, at least one tertiary amine or quaternary ammonium group. Usually the cationic polymers employed in the compositions of the present invention have an average molecular weight between about 1000 and 3,000,000.

Representative cationic polymers which can be used in accordance with the present invention include, especially, the following polymers:

1. The quaternary derivatives of cellulose ether of the formula:

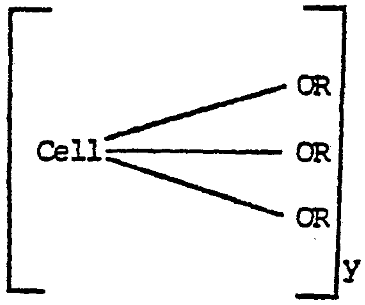

wherein Cell represents the residue of an anhydroglucose unit, y represents a whole number between about 50 and about 20,000, and preferably between about 200 and about 5000, and the radicals R, identical or different, represent a group of the formula:

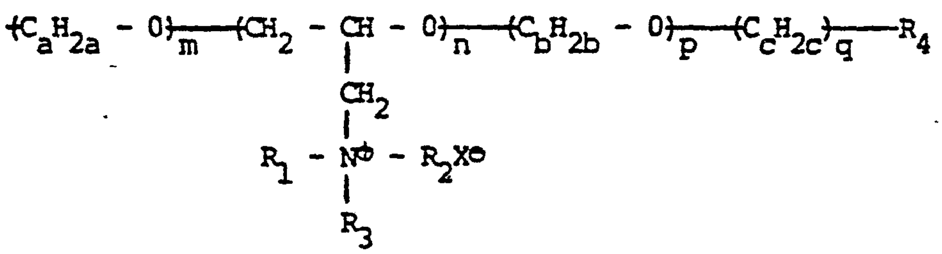

wherein a and b are 2 or 3; c is 1, 2 or 3; m an p are whole numbers from 0 to 10; n is a whole number from 0-3; q is 0 or 1; $R_1$, $R_2$ and $R_3$ each independently represent alkyl, aryl, aralkyl, alkaryl, cycloalkyl, alkoxyalkyl or alkoxyaryl, containing 1-10 carbon atoms and such that the sum of the number of carbon atoms of $R_1$, $R_2$ and $R_3$ varies from 3-12; it being understood that when any one of $R_1$, $R_2$ and $R_3$ is alkoxyalkyl, there are at least two carbon atoms between the oxygen of the alkoxy moiety and the nitrogen atom to which the alkyl moiety is attached; and $X^-$ represents the anionic residue of a mineral or organic acid. $R_4$ is selected from H, 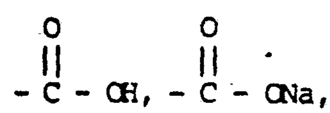

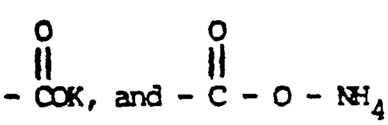, and with the proviso that when q is 0 than $R_4$ is H.

Representative anions designated as $X^{\ominus}$, include, for instance, chloride, bromide, iodide, sulfate, bisulfate ($HSO_4^-$), $CH_3SO_3^-$, sulfonate, phosphate, acetate and the like.

The average value of n is between about 0.01 and 1 per anhydroglucose unit, and preferably between about 0.1 and 0.5.

The average value of (m+n+p+q) is between about 0.01 - 4 per unit of anhydroglucose.

Such quaternary derivatives of cellulose ether are described, for instance, in French Patent 1,492,597 as well as in U.S. Patent No. 3,472,840, incorporated herein by reference.

The quaternary derivatives of cellulose ether can be prepared in accordance with processes described in these two patents, for instance, by etherification and quaternization; these two operations being able to be made in any order or even simultaneously.

The etherification operation effects the fixation on the cellulose chain of a short chain alkyl or hydroxy alkyl substituent having, for example, up to 4 carbon atoms, and preferably an alkyl substituent having 1-3 carbon atoms or a hydroxyalkyl substituent having from 2-4 carbon atoms.

To effect etherification there is employed, preferably, an alkylating agent such as dimethylsulfate, diethyl sulfate, methyl chloride, methyl bromide, ethyl chloride, ethyl bromide or n-propyl chloride; or a hydroxy alkylating agent such as ethylene oxide or propylene oxide.

For the quaternization reaction, there is employed a quaternary halohydrin of the formula:

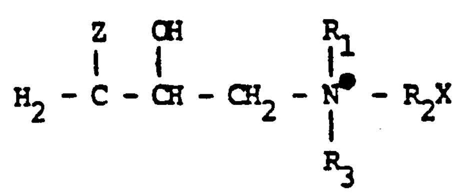

or a quaternary epoxide of the formula

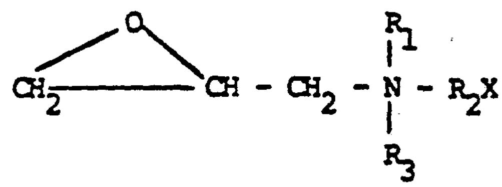

wherein $R_1$, $R_2$ and $R_3$ are defined above, Z is Cl, I or Br, and $X^-$ is an anion and preferably the anion residue of a strong mineral acid.

The radicals R fixed on the anhydroglucose chain can be, for example, the following: H, $-CH_3$, $-C_2H_5$, $-CH_2CH_2OH$, $-(CH_2CH_2O)_s-CH_2OH$ wherein s is 1 or 2.

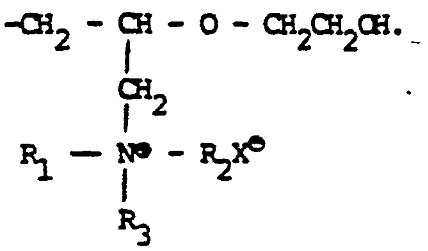

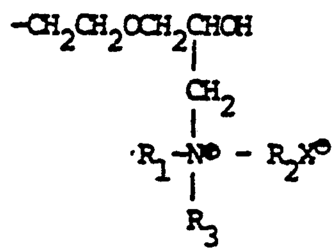

and

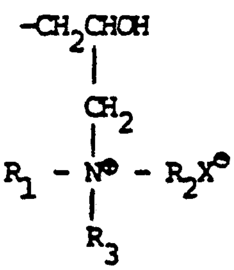

wherein $R_1$, $R_2$ and $R_3$ are defined as above and represent for example methyl or ethyl, with X being, for example, Cl.

When the quaternized polymer of cellulose ether is prepared starting with a cellulose ether, the later is preferably selected from the group consisting of nonionic water soluble cellulose ethers and nonionic water soluble cellulose ethers substituted by a short chain alkyl or hydroxyalkyl. These derivatives are, principally methyl-, ethyl-, or hydroxyethylcellulose.

Representative quaternized derivatives of cellulose ethers usefully employed in the present invention include the commerical product UCARE POLYMER JR sold by Union Carbide Corporation.

2. Copolymers of the formula

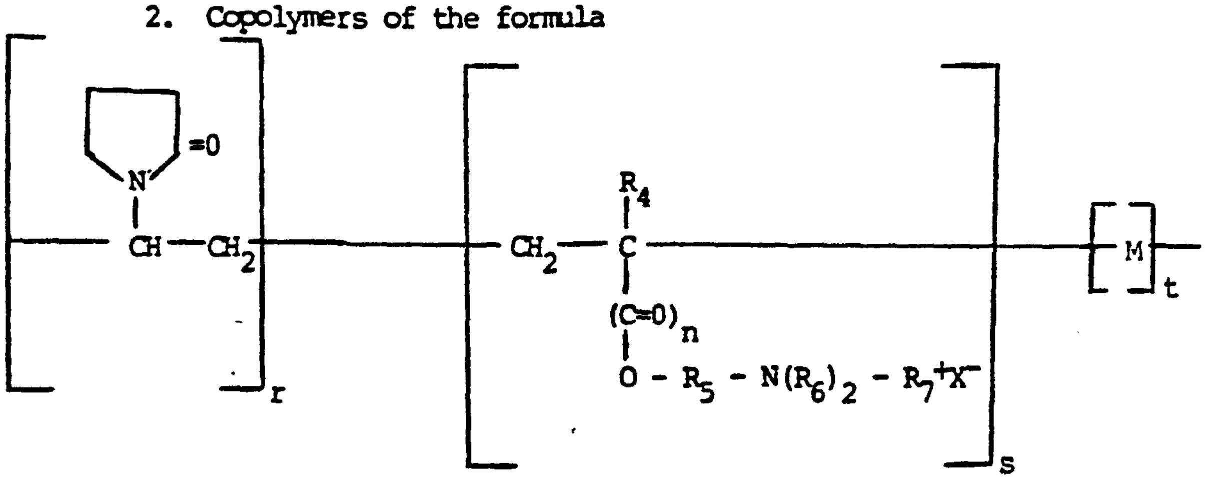

wherein, on the basis of 100 moles of monomer units (i.e. r+s+t = 100), r is a whole number between 20 and 99, s is a whole number between 1 and 80 and t is a whole number between 0 and 50; n = 0 or 1; $R_4$ represents hydrogen or methyl; $R_5$ represents

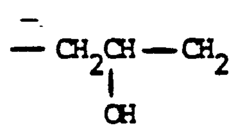

or $C_xH_{2x}$ wherein x is a whole number between 2 and 18; $R_6$ is methyl, ethyl or tert-butyl; $R_7$ is methyl, ethyl or benzyl; $X^-$ represents a chloride, bromide, iodide, sulfate, bisulfate or $CH_3SO_3^-$ anion; and M represents a copolymerizable vinyl monomer unit.

The optionally present copolymerizable vinyl monomer represented by M in the foregoing formula, is a conventional vinyl monomer copolymerizable with N-vinyl pyrrolidone. These vinyl monomers are principally alkyl

vinyl ethers wherein the alkyl moiety has preferably from 1-8 carbon atoms, for example, methyl vinyl ether, ethyl vinyl ether, octyl vinyl ether; alkyl esters of acrylic or methacrylic acid, principally those wherein the alkyl moiety has from 1-4 carbon atoms, for instance, methyl acrylate or methyl methacrylate; vinyl aromatic monomers such as styrene and methyl styrene; vinyl esters such as vinyl acetate; vinylidene chloride; acrylonitrile; methacrylonitrile; acrylamide; methacrylamide; vinyl chloride; and alkyl crotonates, wherein the alkyl moiety has, preferably, from 1 to 8 carbon atoms, and the like.

These copolymers are prepared by copolymerization of (1) N-vinylpyrrolidone, (2) the acrylate or methacrylate of di-lower alkyl-amino alkyl or the acrylate or methacrylate of di-lower alkyl amino hydroxyalkyl; and (3) optionally another vinyl monomer copolymerizable with the vinylpyrrolidone. Taking 100% as the molar basis, the units of vinylpyrrolidone represent from 20-99%, the units attributable to the acrylate or methacrylate represent between 1 and 80% and the units attributable to the other copolymerizable vinyl monomer represent between 0 and 50%.

Representative acrylates or methacrylates usefully employed in the production of such copolymers are principally the following:
dimethylaminomethyl acrylate, dimethylaminomethyl methacrylate, diethylaminomethyl acrylate, diethylaminomethyl methacrylate, dimethylaminoethyl acrylate, dimethylaminoethyl methacrylate, dimethylamino-2-hydroxypropyl acrylate, dimethylamino-2-hydroxypropyl methacrylate, diethylamino-2-hydroxyethyl acrylate, diethylamino-2-hydroxyethyl methacrylate,

dimethylaminobutyl acrylate, dimethylaminobutyl methacrylate, dimethylaminoamyl methacrylate, diethylaminoamyl methacrylate, dimethylaminohexyl acrylate, diethylaminohexyl methacrylate, dimethylaminooctyl acrylate, dimethylaminooctyl methacrylate and diethylaminooctyl acrylate.

The molecular weight of these copolymers is generally between 15,000 and 1,000,000 and more particularly between 50,000 and 500,000.

Representative of such copolymers are copolymers known under the commerical name of GAFQUAT 734 and 755, sold by GAF. The average molecular weight of GAFQUAT 734 is about 100,000 and that of GAFQUAT 755 is greater than 1,000,000.

3. Copolymers of the general formula

- A - Z - A - Z - A - Z -

wherein A represents a radical containing two secondary amine functions, preferably a radical derived from a heterocycle containing two secondary amine functions, e.g. the radical

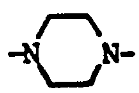

and

Z represents a divalent radical B or B' wherein B and B', identical or different, represent a straight or branched chain alkylene having up to 6 carbon atoms in the principal chain and also able to carry oxygen, nitrogen and/or sulfur atoms and from 1-3 aromatic or heterocyclic rings the oxygen, nitrogen and sulfur atoms can be present in the form of ether, thioether, sulfoxide, sulfone, sulfonium, amine, amine oxide, quaternary ammonium, amide, imide, urea, alcohol, ester and/or urethane groups.

These copolymers can be quaternized by lower alkyl or lower alkyl phenyl groups, principally, methyl, ethyl or benzyl groups.

The quaternization can be effected, for instance, with the aid of known quaternization agents such as, for example, lower alkyl chloride, bromide, iodide, sulfate, mesylate or tosylate or benzyl chloride or bromide.

These copolymers have an average molecular weight generally between 1000 and 15,000.

Representative of these copolymers are, in particular, those obtained by the polycondensation of piperazine and epichlorohydrin. The molar proportion of piperazine to epichlorohydrin can vary, for instance, from 0.5:2 to 2:0.5. These polycondensates can also be quaternized by lower alkyl or lower alkyl phenyl radicals as indicated above.

Such copolymers are described in U.S. Patent No. 3,917,817, incorporated herein by reference.

The liquid skin cleansers herein are preferably in the form of liquids including creams in which water is the principal diluent. The level of water in the compositions is typically from about 60% to about 93% by weight, preferably from 70-85% by weight.

The pH of the liquid skin cleanser compositions herein is in the range of about 4.0 to about 9.0. When it is desired to have the pH of the formulation approximate the pH of skin, the pH is preferably in the range of about 5.0 to about 6.5. Suitable pH adjustment agents include hydrochloric acid, phosphoric acid, succinic acid and sodium citrate/citric acid combinations, among others.

The skin cleansers herein can contain a variety of optional ingredients suitable for improving such compositions in a variety of ways. Such conventional optional ingredients are well known to those skilled in the art, e.g., preservatives such as benzyl alcohol, methyl paraben, propyl paraben, glutaraldehyde, formaldehyde, 3-isothiazolinones

and imidazolidinyl urea can be used in amounts of from 1 to 5,000 ppm; thickeners and viscosity modifiers such as polyethylene glycols, sodium chloride, ammonium chloride, sodium sulfate, carboxymethyl cellulose, methylcellulose, polyvinyl alcohol, and ethyl alcohol; suspending agents such as magnesium/aluminum silicate; perfumes, dyes; opacifiers such as behenic acid, ethylene glycol mono or distearate and calcium stearate; sequestering agents such as the disodium salt of ethylenediamine tetraacetic acid; emollients, moisturizers and various other skin treating ingredients such as glycerin, propylene glycol, and lanolin; buffers and builders such as citrates and phosphates; higher fatty acid alkanolamides such as the diethanolamides, di- or monoisopropanolamides and monoethanolamides of fatty acids having about 10 to 14 carbons in the alkyl radical. Examples are lauric, capric, myristic and coconut mono- and diethanolamides, and mixtures thereof.

The composition of this invention contains from about 5 to about 30 weight percent, preferably from about 10 to about 20 weight percent of the synthetic detergent; from about 0.5 to about 5 weight percent, preferably from about 2 to about 4 weight percent of the fatty acid soap; from about 0.1 to about 2.0 weight percent, preferably from about 0.5 to about 1.0 weight percent of the water soluble cationic polymer, and from about 0.01 to about 10 weight percent of one or more of the optional ingredients.

The liquid skin cleanser composition of this invention may be formulated in several different ways. For example, the water soluble cationic polymer is first dissolved in water and when thoroughly dispersed, preservatives may be added and the solution heated to a temperature of from about 70 to about 80°C. In a second vessel the anionic surfactant and fatty acid are heated to a temperature of from about 70 to about 80°C.

0106193

In a third vessel a base is dissolved in water and heated to a temperature of from about 70 to about 80°C. The products in the third vessel are then added to the second vessel. The solution containing the water soluble cationic polymer is then added to this vessel. The mixture is stirred and then cooled to about 35 to 40°C. The pH of the formulation can then be adjusted to the desired pH. The formulation is then ready for packaging or use.

It is apparent to those skilled in the art that there are different ways of formulating the compositions of this invention. However, it is preferable to prepare the fatty acid soap in situ.

## EXAMPLES

The following examples serve to give specific illustrations of the practice of this invention, but they are not intended in any way to limit the scope of this invention.

### EXAMPLE 1

| | Ingredients | Percent by Weight |
|---|---|---|
| A. | Sodium Laureth-2 Sulfate | 13.00 |
| | Coco Diethanolamide | 3.00 |
| | Lauric Acid | 2.00 |
| B. | UCARE Polymer JR-30M[1] | 0.80 |
| | Imidazolidinyl Urea | 0.20 |
| | Propyl Paraben | 0.05 |
| | Methyl Paraben | 0.10 |
| | Water | 34.65 |
| C. | Sodium Hydroxide (99%) | 0.35 |
| | Water | 7.85 |
| D. | Ethylene Glycol Distearate | 1.00 |

[1]A cationic cellulose resin sold by Union Carbide Corporation. It has a viscosity of 1,000-2,500 centipoise (a one percent solution measured on a ... It is prepared by the methods as

BAD ORIGINAL

The polymer was dissolved in the water of part B. When the polymer was thoroughly dispersed, the preservatives of part B were added, and the solution heated to 70 to 75°C. The ingredients of parts A and C were heated in separate vessels to 70 to 75°C. The ingredients of part C were added to those of part A with stirring. The ingredients of Part B were then added. The ethylene glycol distearate was melted and then added to other ingredients. The stirring was continued until the mixture cooled to about 35 to 40°C. The pH of the formulation was adjusted to 7.5 by the addition of citric acid. The formulation is then ready for packaging or use.

EXAMPLE 2

The procedure of Example 1 was exactly repeated except that the pH was adjusted to 5.5 by the addition of citric acid.

EXAMPLE 3

A liquid cleanser was prepared by the procedure of Example 1 except that the ingredients were the following:

| | Ingredients | Percent by Weight |
|---|---|---|
| A. | Ammonium lauryl sulfate | 13.00 |
| | Coco Diethanoloamide | 2.00 |
| | Lauric Acid | 2.00 |
| B. | UCARE Polymer JR-400[2] | 0.80 |
| | Imidazolidinyl Urea | 0.20 |
| | Propyl Paraben | 0.05 |
| | Methyl Paraben | 0.10 |
| | Water | 34.65 |
| C. | Sodium Hydroxide (99%) | 0.35 |
| | Water | 7.85 |
| D. | Ethylene Glycol Distearate | 1.00 |

The pH was adjusted as in Example 1 to 7.5.

[2] A cationic cellulose resin sold by Union Carbide Corporation. It has a viscosity of 300-500 centipoise (a one percent solution measured or a Brookfield LVF, 30 rpm. spindle speed). The resin is prepared by the methods as described in the Examples of U.S. Patent 3,472,840.

EXAMPLE 4

The procedure of Example 3 was exactly repeated except that the pH was adjusted to 5.5 by the addition of citric acid.

The formulations of Examples 1 and 3, those of pH 7.5, were evaluated in conventional hand washing tests and found to exhibit outstanding cleaning and lathering with no dry after-feel. The formulations of Examples 2 and 4, those of pH 5.5, were also evaluated in the conventional hand washing test and found to exhibit good cleaning and exceptional creamy lathering while imparting a soft, moist after-feel to the skin.

<u>WHAT IS CLAIMED IS:</u>

1. A liquid skin cleanser composition comprising:

(a) a synthetic detergent,

(b) a fatty acid soap,

(c) a water soluble cationic polymer, and

(d) water.

2. A composition as defined in claim 1, wherein the water soluble cationic polymer is a quaternized cellulose derivative.

3. A composition as defined in claim 1, wherein the water soluble cationic polymer is a synthetic polymer containing quaternary ammonium groups.

4. A liquid skin cleanser composition as defined in claims 1 or 2 or 3 wherein the pH is from about 5.0 to about 6.5.